# EUROPEAN PATENT APPLICATION

(11) **EP 4 740 931 A1**
(43) Date of publication of application: **13.05.2026**
(21) Application number: 24211116.9
(22) Date of filing: 06.11.2024
(51) Int. Cl.: A61K 8/34, A61K 8/365, A61K 8/37, A61K 8/41, A61K 8/73, A61K 8/81, A61K 8/9728, A61P 1/02, A61Q 11/00, A61K 8/65, A61K 9/00, A61K 47/10, A61K 47/18, A61K 47/32, A61K 47/36

(54) **ORAL CARE COMPOSITION FOR USE IN THE TREATMENT OF DRY MOUTH**

(71) Applicant: Oral Care Solutions GmbH, 8032 Zurich (CH)
(72) Inventor: ORTIZ SANCHEZ, Virginia Beatrice, 8032 Zurich (CH)
(74) Representative: Calysta NV

(57) **Abstract**

An oral care composition for use in the treatment of dry mouth comprising at least one thickening agent in an amount of 0.1 to 15 wt% based on the weight of the oral care composition, a pH buffering agent in an amount of 0.5 to 2 wt% based on the weight of the oral care composition, at least two humectants in an amount of 15 to 30 wt% based on the weight of the oral care composition, water in an amount of 66.5 to 83.6 wt% based on the weight of the oralcare composition, under the form of a gel.

## Description

The present invention relates to an oral care composition for use in the treatment of dry mouth.

Xerostomia, commonly known as dry mouth, is a condition where the salivary glands don't produce enough saliva to keep the mouth properly moist. This can lead to discomfort and various oral health problems. Dry mouth is typically a symptom of underlying conditions rather than a disease on its own.

The symptoms of xerostomia include a persistent dry and sticky feeling in the mouth and throat, difficulty chewing, swallowing, or speaking, and a burning or tingling sensation in the mouth, particularly on the tongue.

Complications from xerostomia can be significant. Saliva helps to wash away food particles and bacteria while neutralizing acids that can damage tooth enamel, so without it, there is a greater risk of tooth decay. Additionally, the lack of moisture in the mouth can lead to gum disease and oral infections, such as oral thrush (a fungal infection). People with dry mouth may also find it difficult to wear dentures comfortably because there isn't enough natural lubrication in the mouth.

Moisturizing the mouth is more challenging than moisturizing the skin due to differences in environment and tissue types. The mouth's mucous membranes are constantly exposed to saliva, food, and enzymes, making it harder to retain moisture and active ingredients compared to the skin, where creams can form a lasting barrier. Treating the mouth requires non-toxic, biocompatible ingredients because products are often ingested, unlike skin treatments. Additionally, the mucosa is more sensitive and easily irritated, increasing the need for safe, gentle formulations.

Taste is another key factor in oral moisturizers, as any unpleasant flavor can discourage use, unlike skin care products where taste isn't a concern. The product must also dissolve smoothly in the mouth without interfering with eating or speaking, further complicating formulation. In summary, moisturizing the mouth requires products that are safe to ingest, gentle on sensitive tissues, effective at maintaining moisture, and pleasant in taste.

Known oral moisturizers in gel form are developed to manage and treat dry mouth. These products are designed to stimulate temporary saliva production. These gels are formulated with flavors that help activate the salivary glands, encouraging natural saliva flow.

Flavored gels stimulate saliva production by activating taste receptors in the mouth. When applied, the flavors, especially sour or sweet, trigger the salivary glands to produce more saliva. This natural response helps relieve dry mouth by combining the gel's moisture with increased saliva flow. The additional saliva aids in digestion, cleanses the mouth, and helps protect teeth and gums, making these gels an effective treatment for xerostomia.

Some disadvantages can limit the effectiveness of flavored gels designed to stimulate saliva production. One of the main issues is their short-lived efficacy, as the limited increase in saliva production often provides only temporary relief, requiring frequent reapplication to keep the mouth moist. Further, taste receptors are quickly saturated and further intake of the flavored gel does not reactivate the saliva production stimulation because of the saturation of the receptors. Accordingly, the user should delay one intake from another one to keep the saliva production stimulation effect. Additionally, the flavors used in these gels may not be appealing to everyone. Strong or tangy flavors, such as sour or minty ones, can be unpleasant for some users, making it uncomfortable and reducing their willingness to use the product regularly.

There is also the potential for irritation, particularly in individuals with sensitive oral mucosa or allergies to certain ingredients. Over time, repeated exposure to the same flavor can lead to taste fatigue, where users grow tired of the flavor, which may further decrease consistent use. For individuals with severe dry mouth conditions, like advanced cases of Sjogren's syndrome, the gels may not be able to stimulate enough saliva to provide significant relief. These factors collectively make flavored oral gels less suitable or less effective for all users.

Most flavoured oral gels currently used to treat dry mouth contain ingredients with aquatic toxicity, contributing to water pollution. These compounds often include cellulose-based thickening agents, such as carbomethoxy cellulose, hydroxyethyl cellulose, or hydroxymethyl cellulose. Whether users spit out or swallow these gels, they ultimately enter the wastewater system, creating challenges for water treatment plants where such facilities exist. In areas without treatment plants, these toxic substances are directly released into the environment.

The present invention encounters to solve at least a part of these drawbacks by providing an oral care composition offering both immediate and prolonged relief from dry mouth symptoms without harming the environment.

To solve this problem, it is provided according to the present invention, an oral care composition for use in the treatment of dry mouth as mentioned in the beginning, comprising at least one thickening agent in an amount of 0.1 to 15 wt% based on the weight of the oral care composition, preferably 0.5 to 10 wt% based on the weight of the oral care composition, more preferably 0.9 to 5 wt% based on the weight of the oral care composition, a pH buffering agent in an amount of 0.5 to 2 wt% based on the weight of the oral care composition, at least two humectants in an amount of 15 to 30 wt% based on the weight of the oral care composition, water in an amount of 66.5 to 83.6 wt% based on the weight of the oral care composition, under the form of a gel.

It has been indeed surprisingly identified that the aforementioned composition allows an immediate and prolonged relief from dry mouth symptoms. Without being bound precisely to any theory, it is thought that the present invention allows a very good synergy between the thickening agent and the high amount of humectants, which is further improved by the pH buffering agent and that the gel structure of the oral care composition is protected against saliva enzymes, allowing the long lasting effect.

Indeed, pH buffering agent has a double role of stabilizing the gel structure of the oral care composition and maintaining a balanced pH in the mouth, which becomes more acidic when saliva production is reduced. By neutralizing acidity, the buffering agent not only creates a more comfortable environment and stabilize the gel structure but also protects against the harmful effects of a dry, acidic mouth, such as tooth decay and irritation. This, in turn, supports the work of the thickening agent by preventing further damage to the oral tissues, allowing them to heal and retain moisture more effectively.

The thickening agent plays a critical role by giving the product a viscous consistency under a gel form that allows it to coat the oral tissues, forming a protective layer. This barrier retains moisture and ensures that the product remains in contact with the mucosa for an extended period, which is crucial for prolonged relief in a dry mouth environment. However, its effectiveness is greatly amplified when combined with the aforementioned amount of humectants, which broadly and actively attract and retain moisture. While the thickening agent keeps the product in place, the humectants draw water from the surrounding environment or deeper tissues, providing continuous hydration to the oral surfaces. This dual action significantly enhances the moisture retention capabilities of the formulation.

Together, these components create a powerful, synergistic effect. The thickening agent maintains the product's presence in the mouth, the humectants actively hydrate the mucosa, and the pH buffering agent preserves the mouth's natural balance. This holistic approach not only provides immediate relief from dryness but also addresses the long-term health of the oral tissues, making it a highly effective solution for managing xerostomia.

Advantageously, the at least one thickening agent is chosen in the group comprising carbomers, natural gums, modified starch, sodium alginate, pectin, gelatin.

Preferably, each thickening agent is present in an amount of 0.1 to 5 wt% based on the weight of the oral car composition.

The use of carbomer as a thickening agent in cosmetics is well-established, particularly in creams and lotions, where its ability to enhance texture ensures a smooth, spreadable consistency that is ideal for application on the skin. Surprisingly the application of carbomer in the oral care field, particularly for the treatment of dry mouth (xerostomia) provides remarkable results. In oral care formulations, carbomer provides a pleasant and manageable texture in the mouth, improving the overall sensory experience for the user.

Beyond its texture-enhancing qualities, carbomer plays a crucial role in ensuring that the oral care composition adheres to the mucous membranes of the mouth. This binding capacity allows the product to stay in place longer, which is particularly beneficial for individuals suffering from dry mouth. By remaining in contact with the oral tissues, the formulation can provide sustained relief, delivering moisture over an extended period and ensuring a longer-lasting soothing effect. This dual function, enhancing texture and promoting adhesion, makes carbomer an effective ingredient in oral care products designed for moisturizing and treating dry mouth conditions.

Natural gums are plant-based and effectively increase the viscosity of oral gels, creating a smooth texture that adheres well to the oral tissues. This adherence is important for providing prolonged relief from dry mouth. Being of natural origin, these gums are biocompatible, safe for long-term use, and less likely to cause irritation. They also maintain their stability across a wide range of temperatures and pH levels, ensuring consistent performance.

Modified starches offer similar benefits, providing flexibility in adjusting the gel's viscosity while maintaining a smooth, comfortable texture. Starches are non-irritating and easy to tolerate, making them an excellent option for sensitive oral environments. Sodium alginate, derived from seaweed, forms a thick, gel-like structure when in contact with moisture, making it ideal for coating the mucous membranes and providing a lasting barrier against dryness. It is also highly biocompatible and promotes a gentle but effective moisture retention mechanism.

Pectin, commonly derived from fruits, adds both thickness and stability to oral gels. Its natural origin ensures it is safe for use in the mouth, while its ability to form gels in the presence of water helps create a protective layer over the oral tissues. Gelatin, a protein-based thickening agent, offers similar benefits, forming a gel-like consistency that adheres well to the mouth and provides soothing relief for dry areas. It is particularly effective in creating a smooth, hydrating texture.

Preferably, the carbomers are carboxypolymethylene, preferably a Carbopol, more preferably Carbopol 974.

More preferably, said Carbopol is being supplemented with a sufficient amount of the pH buffering agent to be stable at a pH in the range from 4.5 to 7.5.

Preferably, said Carbopol is being supplemented with a sufficient amount of tris(hydroxymethyl)aminomethane to be stable at a pH in the range from 4.5 to 7.5.

The gel is formulated to be stabilized with the pH buffering agent, ensuring optimal stability when applied in the mouth, even under conditions where the oral pH ranges between 4.5 and 7.5. Such low pH values are not typical of normal oral physiology but can occur as a result of dry mouth, which often leads to an acidic environment. The oral pH, influenced by reduced salivary flow, is measured using pH test strips.

Advantageously, the natural gum is chosen in the group comprising arabic gum, xanthan gum, guar gum, carob bean gum, gellan gum, gum tragacanth.

Preferably, the at least one thickening agent comprises 2, 3, 4, 5, 6, or 7 different thickening agents, in a total amount corresponding to the amount of said at least one thickening agent. For example, the at least one thickening agent is Carbopol 974 in an amount of 0.5%wt based on the weight of the oral care composition, Arabic gum in an amount of 0.5%wt based on the weight of the oral care composition and tragacanth in an amount of 0.25%wt based on the weight of the oral care composition. Accordingly, the amount of at least one thickening agent in the aforementioned exemplified composition is the total of each thickening agent, i.e. 1.25 %wt based on the weight of the oral care composition.

Advantageously, the pH buffering agent is tris(hydroxymethyl)aminomethane. Tris(hydroxymethyl)aminomethane helps ensure that products remain at the desired pH for stability and effectiveness. It also neutralizes the composition and allows an alcoholic strength up to 45% V/V. The absence of secondary amines in the pH buffering agent prevents the formation of nitrosamines which are carcinogens.

Advantageously, the oral care composition for use further comprises a chelating agent, preferably sodium ethylenediaminetetraacetic acid. The ethylenediaminetetraacetic acid binds with and neutralizes metal ions (such as calcium or magnesium) in the mouth, which can interfere with the effectiveness of the product but also on the stability of the gel structure. By doing so, chelating agents enhance the performance of other ingredients in the composition and prevent the buildup of tartar/calculus or biofilm, which can worsen in dry mouth conditions due to reduced saliva's natural cleaning ability.

The chelating agent adds another layer of protection by binding with metal ions, like calcium and magnesium, that can promote tartar buildup and biofilm formation in dry mouth conditions. By neutralizing these ions, the chelating agent enhances the overall efficacy of the formulation, ensuring that the thickening and hydrating effects are not compromised by mineral buildup.

Preferably, the chelating agent is present in an amount from 0.05 to 0.5 wt% based on the weight of the oral care composition, and wherein water is present in an amount of 67 to 83.55 wt% based on the weight of the oral care composition.

Advantageously, the at least two humectants are chosen in the group comprising hyaluronic acid, propylene glycol, glycerin, xylitol, sorbitol, polyethylene glycol, lactic acid, mono and di glyceride, tremella fuciformis, citric acid.

Hyaluronic acid works by attracting and retaining moisture, helping to keep the oral tissues hydrated for longer periods. It provides immediate and lasting relief from discomfort. The hydrating effect of hyaluronic acid helps to soothe the irritation that often accompanies dry mouth, forming a protective film over the mucous membranes. This film reduces friction and discomfort when talking, eating, or swallowing.

Preferably, the at least two humectants are a mixture of hyaluronic acid, xylitol, propylene glycol and glycerin. For example, the at least two humectants is hyaluronic acid in an amount of 0.2%wt based on the weight of the oral care composition, xylitol in an amount of 2.5%wt based on the weight of the oral care composition, propylene glycol in an amount of 10%wt based on the weight of the oral care composition and glycerin in an amount of 8.5%wt based on the weight of the oral care composition. Accordingly, the amount of at least two humectants in the aforementioned exemplified composition is the total of each humectant, i.e. 22.5 %wt based on the weight of the oral care composition.

More preferably, the oral car composition for use further comprises one or more additive, which can be an aroma, a preservative and/or a plant extract. The total amount of additive is comprised between 0.1 and 5%wt based on the weight of the oral care composition, preferably between 0.5 and 2.5 %wt based on the weight of the oral care composition. The additive is preferably chosen in the group comprising chamomile extract, ratanhia extract, clove essential oil, aloe vera, licorice root extract, calendula extract, peppermint extract, sage extract, slippery elm, marshmallow root extract, green tea extract, honey extract, lemon extract, citric acid, sorbic acid, parabens, potassium sorbate, phenoxyethanol, benzyl alcohol, sorbitan caprylate, caprylyl glycol, 1,2-hexanediol, sodium benzoate, chlorhexidine, sodium methylparaben. For example, the aroma, the preservative and/or the plant extract is chamomile extract in an amount of 1 %wt based on the weight of the oral care composition. In another example, the aroma and/or the plan extract is ratanhia extract in an amount of 1.25%wt based on the weight of the oral care composition and clove essential oil in an amount of 0.1%wt based on the weight of the oral care composition. Accordingly, the amount of the additive in the aforementioned exemplified composition is the total of each additive, i.e. 1 %wt and 1.35 %wt based on the weight of the oral care composition. The use of citric acid, sorbic acid and/or parabens reduce the oxidation of the oral care composition resulting in a long shelf life and enhanced durability.

Advantageously, the oral care composition for use is packaged in a liquid single dose sachet.

Single-dose sachets are highly portable and easy to use, making them ideal for individuals who suffer from dry mouth and need quick, on-the-go relief. The sachet format provides a pre-measured amount of gel, ensuring consistent dosing without the need for guesswork, which enhances the overall user experience by providing the right quantity every time.

Another significant advantage is the improved hygiene. Each sachet is individually sealed, reducing the risk of contamination that can occur with larger, multi-use containers. This single-use format also minimizes the exposure of the product to air and bacteria, keeping it sterile and safe for use, particularly in sensitive oral environments.

The liquid form within the sachet makes the gel easy to apply directly onto the oral tissues, allowing it to spread quickly and evenly in the mouth. The gel's texture ensures that it adheres to the mucous membranes for long-lasting moisture and relief, while the single-dose format encourages efficient use without waste.

Additionally, single-dose sachets offer practicality for night-time or daily use, ensuring that users can easily carry them and apply them whenever necessary, providing a simple, hygienic, and effective way to manage dry mouth symptoms.

Preferably, the liquid single dose sachet is packaged with a series of liquid single dose sachet in a box.

Other embodiments according to the present invention are mentioned in the appended claims.

The present invention also aims to provide a method to improve oral health by treating dry mouth.

It is also known that improving oral health by treating dry mouth focuses on restoring moisture, stimulating saliva production, and protecting the oral environment. Regular hydration, along with the use of oral gels or sprays helps maintain moisture in the mouth. Saliva production can be stimulated by chewing sugar-free gum or using medications when necessary. Maintaining a balanced pH with buffering agents protects tooth enamel and prevents decay. Additionally, oral care products with soothing ingredients help repair and protect tissues affected by dryness.

Oral gels work as a treatment for dry mouth by providing moisture and forming a protective layer over the oral tissues. The gel contains ingredients like humectants, which attract and retain water, keeping the mouth hydrated. It adheres to the mucous membranes, offering long-lasting relief from dryness. By maintaining moisture and reducing irritation, oral gels effectively alleviate the discomfort associated with dry mouth and support better oral health.

The gel consistency in oral products is obtained by using thickening agents. These agents are added to the formulation to increase viscosity, giving the product its gel-like structure. When mixed with water or other liquids, these thickening agents form a semi-solid, smooth texture that allows the gel to adhere to surfaces, like the oral mucosa, and stay in place longer. This consistency is essential for providing sustained moisture and comfort in the treatment of dry mouth, as it ensures the gel remains in contact with the tissues.

The method according to what has been mentioned before is characterized in that it comprises applying an effective amount of an oral care composition described above to the oral cavity of a subject in need thereof, wherein the method is effective to treat dry mouth.

Other characteristics and advantages of the present invention will be derived from the non-limitative following description, and by making references to the drawings and the examples.

### Examples.-

### Example 1

To produce an oral care composition to use according to the invention, the liquid components are mixed first: 77,98g of water was mixed with 9.97g of glycerin 85% in water and 9.99g of propylenglykol. Then the solid components are mixed: 1g of Carbopol 974 was mixed with 1g of tris(hydroxymethyl)aminomethane and 0.1g of sodium ethylenediaminetetraacetic acid. The solid mixture is then added to the liquid mixture. The gel production is made by prolonged stirring for more than 30 minutes and minimal air exposure.

### Example 2

Following the same procedure as example 1, using 2.5g of xylitol, 10g of propylene glycol, 10g of glycerin 85% in water, 74.45g of water to make the liquid mixture and 0.2g of sodium hyaluronate, 0.5g of Carbopol 974, 0.5g of tris(hydroxymethyl)aminomethane, 1g of chamomile extract, 0.5g of Arabic gum, 0.25g of tragacanth, 0.1g of sodium ethylenediaminetetraacetic acid to make the solid mixture.

It should be understood that the present invention is not limited to the described embodiments and that variations can be applied without going outside of the scope of the appended claims

## Claims

1. An oral care composition for use in the treatment of dry mouth comprising at least one thickening agent in an amount of 0.1 to 15 wt% based on the weight of the oral care composition, preferably 0.5 to 10 wt% based on the weight of the oral care composition, more preferably 0.9 to 5 wt% based on the weight of the oral care composition, a pH buffering agent in an amount of 0.5 to 2 wt% based on the weight of the oral care composition, at least two humectants in an amount of 15 to 30 wt% based on the weight of the oral care composition, water in an amount of 66.5 to 83.6 wt% based on the weight of the oral care composition, under the form of a gel.

2. The oral care composition for use according to claim 1, wherein the at least one thickening agent is chosen in the group comprising carbomers, natural gums, modified starch, sodium alginate, pectin, gelatin.

3. The oral care composition for use according to claim 2, wherein the carbomers are carboxypolymethylene, preferably a Carbopol, more preferably Carbopol 974.

4. The oral care composition for use according to claim 2 or 3, wherein the natural gum is chosen in the group comprising arabic gum, xanthan gum, guar gum, carob bean gum, gellan gum, gum tragacanth.

5. The oral care composition for use according to any of the preceding claims, wherein said at least one thickening agent comprises 2, 3, 4, 5, 6, or 7 different thickening agents, in a total amount corresponding to the amount of said at least one thickening agent.

6. The oral care composition for use according to claim 1 or 2, wherein the pH buffering agent is tris(hydroxymethyl)aminomethane.

7. The oral care composition for use according to any of the preceding claims, further comprising a chelating agent, preferably sodium ethylenediaminetetraacetic acid.

8. The oral care composition for use according to claim 5, wherein the chelating agent is present in an amount from 0,05 to 0,5 wt% based on the weight of the oral care composition, and wherein water is present in an amount of 67 to 83.55 wt% based on the weight of the oral care composition.

9. The oral care composition for use according to any of the preceding claims, wherein the at least two humectants are chosen in the group comprising hyaluronic acid, propylene glycol, glycerin, xylitol, sorbitol, polyethylene glycol, lactic acid, mono and di glyceride, tremella fuciformis.

10. The oral care composition for use according to claim 9, wherein the at least two humectants are a mixture of hyaluronic acid, xylitol, propylene glycol and glycerin.

11. The oral care composition for use according to any of the preceding claims, further comprising one or more additive, which can be an aroma, a preservative and/or a plant extract, preferably chosen in the group comprising chamomile extract, ratanhia extract, clove essential oil, aloe vera, licorice root extract, calendula extract, peppermint extract, sage extract, slippery elm, marshmallow root extract, green tea extract, honey extract, lemon extract, citric acid, sorbic acid, parabens, potassium sorbate, phenoxyethanol, benzyl alcohol, sorbitan caprylate, caprylyl glycol, 1,2-hexanediol, sodium benzoate, chlorhexidine, sodium methylparaben.

12. The oral care composition for use according to any of the preceding claims, packaged in a liquid single dose sachet.

13. The oral care composition for use according to claim 12, wherein the liquid single dose sachet is packaged with a series of liquid single dose sachet in a box.

14. A method to improve oral health comprising applying an effective amount of the oral care composition described in any of the claims 1 to 17 to the oral cavity of a subject in need thereof, wherein the method is effective to treat dry mouth.
